# EUROPEAN PATENT SPECIFICATION

(11) **EP 1 888 069 B1**
(45) Date of publication and mention of the grant of the patent: **02.12.2009**
(21) Application number: 06763171.3
(22) Date of filing: 17.05.2006
(51) Int. Cl.: A61K 47/48, A61K 31/728, A61P 35/00

(54) **ANTIPROLIFERATIVE DRUG**
PROLIFERATIONSHEMMENDES ARZNEIMITTEL
MEDICAMENT ANTIPROLIFERATIF

(30) Priority: 18.05.2005 IE 20050328
(43) Date of publication of application: 20.02.2008
(73) Proprietor: Eurand Pharmaceuticals Ltd, Southern Cross Road Bray, Co. Wicklow (IE)
(72) Inventor: MURANO, Erminio, I-34139 Trieste (IT); FLAIBANI, Antonella, I-33100 Udine (IT); BERGAMIN, Massimo, Trieste, 34139 (IT); NORBEDO, Stefano, I-34135 Trieste (IT); SORBI, Claudia, I-41043 Formigine (IT); KHAN, Riaz Ahmed, Sonning, Berkshire RG4 6TA (GB)
(74) Representative: Gerli, Paolo
(86) International application number: PCT/EP2006/062388
(87) International publication number: WO 2006/122954

(56) References cited:
- EP-A- 1 710 257
- EP-A- 1 724 287
- WO-A-01/68105
- JP-A- 9 188 705
- JP-A- 2004 018 750
- UEKI M. AND INAZU I.: "Synthesis of peptides containing hxdroxiamino acids by the mixed anhxdride method without protecting the hydroxyl functions" CHEMISTRY LETTERS, no. 1, 1982, pages 45-48, XP002409526

## Description

### Field of the invention

The present invention relates to esterified conjugates of hyaluronic acid having anti-proliferative activity.

### Prior art

Several organic molecules have been tested and are known to be useful as antiproliferative agents, but many of them have a limited clinical use due to their high toxicity, low solubility and/or unsuitable pharmacokinetic parameters.

Among these compounds are the N-derivatives of glutamic acids with inhibition activity on dihydrofolate reductase (DHFR), that constitute a family of antiproliferative agents having methotrexate as a parent compound.

These drugs are effective in several pathologies such as neoplasms, psoriasis and rheumatoid arthritis. However, their therapeutic use is strongly limited by their high systemic toxicity.

In WO01/68105 it has been found that the problem of the toxicity of the above DHFR inhibitors can be solved by conjugating them with polysaccharides. In fact, the conjugates thus obtained are still endowed with an antiproliferative activity but, at the same time have the advantage of showing a surprisingly lower toxicity.

JP 09188705 discloses a conjugate comprising hyaluronic acid linked via an amide bond to one of the carboxy-groups of methotrexate.

JP 2004018750 discloses conjugates obtained by coupling of hyaluronic acid amide groups with methotrexate carboxy-groups.

### Summary of the invention

The present inventors have now found that when the conjugates between hyaluronic acid and N-derivatives of glutamic acid described in WO01/68105, are further esterified on the residual primary hydroxyl groups of the hyaluronic acid with an aminoacid, a peptide, an aliphatic acid, an **arylaliphatic** acid and/or arylic acid a significant and unexpected increase in their antiproliferative activity and decrease in their toxicity is observed.

### Description of the figures

Figure 1 represents the ¹³C-NMR spectrum of HA-6-Br obtained in Example 4
Figure 2 represents the ¹³C-NMR spectrum of HA-Cl obtained in Example 5.
Figure 3 represents the ¹³C-NMR spectrum of HA-MTX obtained in Example 19.

### Detailed description of the invention

A first object of the present invention are new conjugates of hyaluronic acid wherein the primary hydroxyl groups of the N-acetyl-D-glucosamine unit of hyaluronic acid are esterified both with the carboxylic group of a compound A selected from the group consisting of aminoacids, peptides, aliphatic acids, aryl-aliphatic acids and arylic acids and with the α- or γ- carboxylic group of a N-derivative of glutamic acid of formula (I): 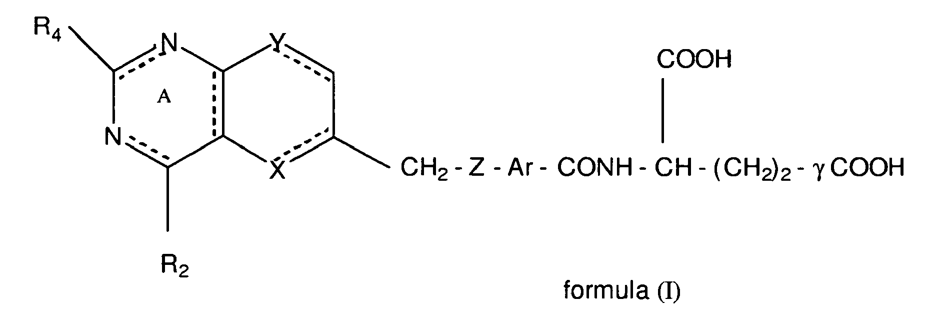 wherein:
R₂ and R₄, independently from one another, represent: -NH₂, -OH, -OCH₃, C₁-C₅ alkyl, =O;
X and Y represent: -C(R₅)=, -CH(R₅)-, -NH-, -N=, wherein R₅ represents: -H, C₁-C₅ alkyl;
Z represents: -CH(R₁₀)-, -N(R₁₀)-, -O-, wherein R₁₀ represents: -H, C₁-C₅ alkyl, C₁-C₅ alkenyl, C₁-C₅ alkynyl, a 5-6 membered heterocyclic ring with 1-3 heteroatoms selected in the group consisting of nitrogen, sulphur and oxygen;
Ar represents: 1,4-phenyl group, 1,4-phenyl group condensed with one or more 5-6 membered aromatic rings, 1,4-phenyl group condensed with one or more 5-6 membered heterocycles, wherein said groups are possibly substituted with R₂ as defined above,
rings A and B, indipendently from one another, may be aromatic or non-aromatic.

These compounds have already been described in WO01/68105.

Preferably, the compound of formula (I) is methotrexate (MTX), represented by formula (I) where R₂ and R₄ are -NH₂; ring A is aromatic; ring B is aromatic and X and Y are: -N=; Z is -N(CH₃)-; Ar is a 1,4-phenyl group.

Further preferred compounds of formula (I) are those belonging to the following subclasses:
- subclass 1 represented by formula (I) wherein R₂ and R₄ are -NH₂ or -OH;
   R₅ when present represents: -H, -CH₃; ring A is aromatic; Z is selected in the group consisting of: -CH(R₁₀)-, -N(R₁₀)-; R₁₀ represents: -H, C₁-C₅ alkyl, C₁-C₅ alkenyl, C₁-C₅ alkynyl.
- subclass 2 represented by formula (I) wherein R₂ is =O and R₄ is -NH₂; ring A is not aromatic; ring B is aromatic and X and Y are -N=; Z is -N(R₁₀)-, wherein R₁₀ represents: -H or -CH₃; Ar is 1,4 phenyl.
- subclass 3 represented by formula (I) where R₂ and R₄ are -NH₂; ring A is aromatic; ring B is aromatic and X and Y are -N=; Z is -N(R₁₀)-; wherein R₁₀ is - CH₃ or-H; Ar is 1,4 phenyl.
- subclass 4 represented by formula (I) wherein R₂ and R₄ are -NH₂; ring A is aromatic; ring B is aromatic and X and Y are -N=; Z is -CH-(C₂H₅)-; Ar is 1,4-phenyl.

The compound of formula (I) is linked to the hyaluronic acid either through its Y- or the α- carboxylic acid group. Compound A is selected from the group consisting of aminoacids, peptides, aliphatic acids, aryl-aliphatic acids and arylic acids and it is linked to the hyaluronic acid through its carboxylic group. This compound is introduced onto the polysaccharide by using the acyl-nucleophile deriving from the corresponding carboxylic acid.

Aliphatic acids according to the present invention are selected from the group consisting of acetic acid, butyric acid, propionic acid, retinoic acid, n-propylacetic acid, succinic acid, cyclohexanecarboxylic acid, cyclohexaneacetylic acid and cyclopropanecarboxylic acid.

Aminoacids according to the invention are selected from the group consisting of alanine, valine, leucine, isoleucine, methionine, glycine, serine, cysteine, asparagine, glutamine, aspartic acid, glutamic acid, proline, histidine phenylalanine, triptophane and tyrosine.

Aryl-aliphatic acids according to the present invention are selected from the group consisting of phenylacetic acid, phenoxyacetic acid, naphtylacetic acid, 2-(4-isobutylphenyl)propionic acid, 2-(6-methoxy-2-naphtyl) propionic acid and cinnamic acid.

Arylic acids are selected from unsubstituted or substituted benzoic acid. Preferably, said substituted benzoic acid is selected from the group consisting of halobenzoic acid, alkylbenzoic acid, nitrobenzoic acid and 2-acetoxybenzoic acid.

The aliphatic or aryl-aliphatic or arylic acid may be substituted with linear or branched C₁-C₅ alkyls, halogens, nitro groups, cyano groups, hydroxyl groups, amino groups, methoxyl groups.

According to a particularly preferred embodiment of the present invention compound A is selected from the group consisting of acetic acid, butyric acid, alanine, glycine and peptides containing alanine and/or glycine.

Hyaluronic acid (also indicated in this application as HA) is composed of a disaccharidic repeating unit, consisting of D-glucuronic acid and 2-acetamido-2-deoxy-D-glucose (N-acetyl-D-glucosamine) bound by β(1→ 3) glycosidic linkage; the D-glucuronic acid residue may either be in the acid form or in the form of a salt. Each repeating unit is bound to the next one by a β(1→4) glycosidic linkage that forms a linear polymer.

The term "hyaluronic acid", as used in the present application, encompasses both the acid and the salified form.

The term hyaluronic acid is commonly used to describe a general group of molecular fractions of HA with varying molecular weights or also hydrolysed fractions of said compound. For the purposes of the present invention the hyaluronic acid has preferably an average molecular weight comprised between 5 000 and 100 000, more preferably between 10 000 and 50 000.

Preferred conjugates of hyaluronic acid of the present invention are 6-O-acetyl-6-O-methotrexyl-hyaluronic acid and 6-O-Butyryl-6-O-methotrexyl-hyaluronic acid and salts thereof.

The conjugates of the invention are either in the acid form or in the salt form. When they are in salt form they may be salified with alkaline metals (preferably Na or K), earth-alkaline metals (preferably Ca or Mg), transition metals (preferably Cu, Zn, Ag, Au, Co, Ag). The salification is obtained by processes known by the skilled artisan.

Optionally, also the secondary hydroxyl groups on the conjugates of the invention may be derivatised to form a group selected from: -OR, -OCOR, -SO₂H, -OPO₃H₂, -O-CO-(CH₂)ₙ-COOH, -O-(CH₂)ₙ-OCOR, wherein n is 1-4 and R is C₁-C₁₀ alkyl.

Alternatively, the secondary hydroxyl groups may be substituted by a -NH2 or - NHCOCH₃ group.

These substitutions can be easily obtained by processes known in the art, and they may be chosen in order to modulate the hydrophilic character of the conjugates.

The total amount of compound of formula (I) and of compound A in the conjugate is defined by the "degree of substitution" (DS_{%}), which indicates the % by weight of the compound of formula (I) and of compound A, with respect to the total weight of the conjugate.

In particular, in the conjugates of the invention the amount of the compound of formula (I) and of compound A (DS%) is preferably comprised between 0.1% and 60% w/w with respect to the total weight of the conjugate. Furthermore, the compound of formula (I) is preferably present in an amount ranging from 1% to 40% w/w, while the compound A is preferably in an amount ranging from 0.1 to 30% w/w, both with respect to the total weight of the conjugate.

Where compound A is acetic acid this is preferably present in the conjugate of the invention in an amount ranging from 0.1% to10% w/w with respect to the total weight of the conjugate.

The conjugates according to the instant invention are characterised by the presence of two different ester groups directly linked to the primary hydroxyl groups of the *N*-acetyl-D-glucosamine units of the hyaluronic acid.

No other hydroxyl groups of the HA are involved in the chemical linkage with the drug, thus providing a regularity in the chemical structure which is highly important to ensure the efficacy.

As demonstrated by the experimental evidence provided herewith, the conjugates of the invention retain the ability of the compounds of formula (I) to inhibit cell proliferation. Furthermore, unexpectedly these derivatives are endowed with a much more potent antiproliferative activity and with a even lower toxicity compared to the corresponding conjugates described in WO01/68105.

Therefore, they can be successfully used in the treatment of all pathologies that are characterised by cell hyperproliferation.

Accordingly, it is a further object of the present invention the use of the above conjugates in the manufacture of a medicament for the treatment of pathologies characterised by cell hyperproliferation. Preferably said pathologies are selected from the group consisting of tumours, skin disorders, psoriasis, inflammatory pathologies and rheumatoid arthritis.

Preferably said tumours are selected from leukaemia, carcinoma (for example adenocarcinoma, colorectal carcinoma, pancreatic carcinoma), breast cancer, ovarian cancer and gastrointestinal tumours.

It is also an object of the present invention a pharmaceutical composition containing the conjugates of the invention in admixture with pharmaceutically acceptable excipients and/or diluents. The pharmaceutical composition may be either in the liquid or in solid form; it may be administered through the oral, parenteral, rectal, or topical route.

A further object of the invention is a process for the preparation of the conjugates described above.

In particular, the present inventors have developed a specific process that allows to obtain the conjugates of the invention devoid of halogenation by-products as well of other by-products that may render them unsuitable for pharmaceutical use.

Said process comprises the following steps, carried out in the order indicated:
(a) chlorination of the primary hydroxyl groups of the N-acetyl-D-glucosamine units of hyaluronic acid either in free or salt form;
(b) formation of ester linkages between the chlorinated hyaluronic acid and the carboxyl groups of the compound of formula (I) by displacement of chlorine atoms;
(c) formation of ester linkages between the product of step (b) and the carboxyl groups of compound A by displacement of the residual chlorine atoms by a suitable acyl nucleophile of the compound A.

The starting HA may be in free form or in the form of salt, wherein the counterion is preferably an alkaline or alkaline-earth metal or is a nitrogen-containing counterion. In the latter case the counterion may contain heterocycles. Preferred examples of nitrogen-containing counterions are ammonium, tetrabutylammonium (TBA), pyridinium or *sym*-collidinium ions.

Step (a) is a selective chlorination which is carried out after mixing the HA in an aprotic organic solvent. It is essential that the reaction in step (a) is a chlorination reaction instead of a generic halogenation reaction.

In fact, as demonstrated in the Experimental part, the chlorination reaction allows to obtain a final conjugate which is stable and free of undesired by-products and impurities that can be harmful to its practical pharmaceutical use.

The preferred chlorination reagent is methanesulphonyl chloride in *N,N-*dimethylformamide (Vilsmeir Reagent).

The chlorination reaction is preferably carried out according to the following procedure.

The chlorinating reagent is added to a solution or suspension of HA in salt form (either sodium form or in an organic base form such as TBA, pyridine or *sym-*collidine), preferably in the sodium form in *N,N*-dimethylformamide (DMF) at temperature ranging from -20°C to -10°C, preferably at -10°C. The reaction temperature is raised from -10°C to between 40°-65°C, preferably 60°C, over a period of 2 h.

The chlorination reaction is then performed at temperature between 40°C and 65°C, preferably at 60°C, for a period of time comprised between 10 and 24 hours, preferably for 16 h.

The reaction is worked up by treatment with saturated aqueous NaHCO₃ solution to achieve pH 8 and then by treatment with aqueous NaOH to pH 9; this step allows to remove the formate ester groups formed during the reaction at the secondary hydroxyl groups of the HA molecule. The reaction mixture is then neutralised by addition of dilute HCl. The desired 6-chloro-6-deoxy-hyaluronic acid (also termed as HA-6-Cl or HA-Cl) is then recovered by means of standard techniques.

Under these conditions the degree of chlorination at the C-6 position of the N-acetyl-D-glucosamine residue could range from 1.0 to 8.3 % w/w, the preferred range being 1.4- 5.0% w/w.

Step (b) is usually performed according to the following procedure.

HA-6-Cl is suspended either in TBA or in sodium salt form, preferably in TBA form, in an aprotic organic solvent or mixture thereof followed by addition of the compound of formula (I) in the same solvent in presence of an alkaline or alkaline-earth metal salt, e.g. cesium carbonate.

The reaction is carried out between 50°C and 80°C, preferably 70°C under constant stirring in the range of 24-52 hours, preferably for 40 hours. The desired product is then recovered by means of standard techniques.

When the compound of formula (I) is MTX the conjugate is called 6-deoxy-6-O-methotrexyl-hyaluronic acid (HA-6-MTX or HA-MTX).

The product obtained in this step is an intermediate conjugate of HA which contains both the compound of formula (I) and chlorine. In this conjugate the total amount of compound of formula (I) is lower than 40% w/w and the residual chlorine (chlorine that has not been displaced during this step) is lower than 3% w/w.

Step (c) allows the complete displacement of the residual chlorine, in the product obtained in the step (b), by an acyl nucleophile of the compound A.

Any suitable acyl nucleophile in alkaline or alkaline-earth metal salt form can be used. In case of acetic acid, butyric acid, or an amino acid, their sodium or cesium salt are preferred. Sodium acetate is preferably used when the (b) product is in the form of sodium salt; whereas the cesium acetate is preferred for the displacement of chlorine from the (b) product in the form of an organic base such as sym-collidinium or TBA salt, preferably in TBA form.

The obtained conjugate may be then recovered by means of standard techniques such as precipitation, ultrafiltration, drying, or freeze-drying.

This conjugate is free from any residual chlorine.

When compound of formula (I) is methotrexate, then the conjugate is 6-O-acyl-6-O-methotrexyl-hyaluronic acid (HA-6-MTX-6-Acyl or HA-MTX-Acyl).

As demonstrated in the experimental section, the present inventors have also surprisingly found that the above process also allows to obtain the conjugate of WO01/68105 containing less that 3% of residual chlorine and therefore endowed with improved properties compared to that prepared with the process described in the prior art.

Accordingly, the present invention also refers to a conjugate between hyaluronic acid and a compound of formula (I), as defined above containing less than 3% w/w and preferably less than 0.1% w/w of residual chlorine chemically linked to HA polymeric chain.

The present invention also relates to a process for obtaining the above conjugate containing less than 3% w/w of halogenation by-products comprising the following steps:
(a) chlorination of the primary hydroxyl groups of the *N*-acetyl-D-glucosamine units of the hyaluronic acid either in free or salt form; and
(b) formation of ester linkages between the chlorinated hyaluronic acid and the carboxyl groups of the compound of formula (I) by displacement of the chlorine atoms;
wherein step a) and step b) are as defined above.

Another object of the present invention is a process for obtaining a conjugate between hyaluronic acid and a compound of formula (I), as defined above containing less than 0.1% w/w of residual chlorine chemically linked to the HA polymeric chain comprising the following steps:
a) chlorination of the primary hydroxyl groups of the *N*-acetyl-D-glucosamine units of the hyaluronic acid either in free or salt form;
(b) formation of ester linkages between the chlorinated hyaluronic acid and the carboxyl groups of the compound of formula (I) by displacement of chlorine atoms;
(c) displacement of the residual chlorine atoms with formation of ester linkages with the residual product of step (b) by a suitable acyl nucleophile; and
d) selective deacylation of the conjugates obtained from step (c).

The reaction can be carried out either by known chemical deacylation reactions or enzymatic deacylation reactions.

The invention is now described in a non-limitative way by the following experimental examples.

### EXPERIMENTAL PART

### EXAMPLE 1: Determination of chlorine content in HA-Cl and HA conjugates

The determination of chlorine content in the conjugate was carried out by ¹³CNMR (Spectrometer Varian Mercury 200) adopting a standard sequence for ¹³C-spectrum acquisition (*std* ¹³C sequence). 20 mg of conjugate sample were dissolved in 650 µL D₂O in a 5 mm tube at room temperature. Mild heating was used (50°C) to eliminate air bubbles in the solution. The spectrum was collected after 24 hr at 30°C and analysed by integrating the CH₂OH signal (61 ppm) and the CH₂Cl signal (44 ppm) for HA-CI. For HA-MTX-Cl derivatives in addition to these two signals, integration was carried out also for CH₂OMTX signal (64 ppm). Chlorine content was determined as a ratio between of the number of HA repeating units containing 6-chlorine groups and the total number of HA repeating units. This ratio was then converted in % by weight of chlorine.

### EXAMPLE 2: Determination of methotrexate content by HPLC

Methotrexate content of HA conjugates was determined by means of HPLC by analysing the samples before and after alkaline hydrolysis according to Methotrexate Official Monograph (USP 23-p 984). The analysis conditions were: Cromatograph: Dionex DX-600. Column: Column Phenomenex Synergi 4µ Hydro-RP80, Column size:150X460mm, Column particle size: 4µ, Temperature: 40°C Eluent: 90% 0.2M dibasic sodium posphate/0.1 M citric acid (630:270), 10% CH₃CN, isocratic condition: 0.5 mL/min. Detector: Diode Array (range 200-780nm), Selected wavelength for the quantitative determination: 302 nm injected volume:25 µl, run time 30 minutes. Solutions for free methotrexate determination were prepared by dissolving HA-MTX directly in MilliQ water at the appropriate concentration. Total methotrexate content was determined after alkaline hydrolysis carried out in NaOH 0.1 M, room temperature for 2 hours. After neutralization with hydrochloric acid 1 M, solutions were filtered through 0.45 µm (Sartorius Minisart RC25 17795Q) prior to injection in the HPLC system. A calibration curve was determined by using standard solutions with known concentration of methotrexate. The method gives the MTX concentration in the sample solution, which normalized by the sample concentration yields the DS_{MTX} %w/w.

The structure of the HA-MTX conjugate was supported by NMR: ¹H-NMR and ¹H-DOESY NMR spectra confirmed the covalent linkage of MTX molecule on C6 position of N-acetyl-D-glucosamine for all conjugates prepared.

### EXAMPLE 3: Determination of weight average molecular weight (Mw)

The molecular weight of the hyaluronic acid conjugates was measured by HP-SEC (High Performance Size Exclusion Chromatography). The analysis conditions were: Cromatograph: HPLC pump 980-PU (Jasco Ser. No. B3901325) with Rheodyne 9125 injector. Column: TSK PWxl (TosoBioscience) G6000+G5000+G3000 6, 10, 13 µm particle size; Temperature: 40°C Mobile phase: NaCl 0.15 M + 0.01% NaN₃. Flux: 0.8 mL/min. Detector: MALLS (WYATT DAWN EOS - WYATT, USA), λ= 690 nm, (dn/dc = 0.167 mL/g), UV spectrophotometric detector 875-UV (Jasco, Ser. No. D3693916), A = 305 nm, Interferometric Refractive Index OPTILAB REX (WYATT, USA); λ=690 nm, Sensitivity: 128x; Temperature: 35°C; injected volume:100 µl, run time 60 minutes. The samples of HA-Cl and of HA-MTX to be analysed were solubilised in 0.9 % NaCl at the concentration of about 1.0 mg/ml and kept under stirring for 12 hours. Then, the solutions were filtered on a 0.45 µm porosity filter (Sartorius Minisart RC25 17795Q) and finally injected in the chromatograph. The analysis allows the measurement of Mw (weight average molecular weight), Mn (number average molecular weight), PI (polydispersity). The concentration of the polymeric samples solutions were controlled by means of the integral of the refractive index.

### EXAMPLE 4: 6-Bromo-6-deoxy-hyaluronic acid (HA-Br)

1 g of tetrabutylammonium salt of hyaluronic acid (MW 120000) was suspended in 50 mL of anhydrous DMF at 80ºC and kept under mixing and under nitrogen atmosphere for ca 1 hour. The mixture was cooled down to room temperature and then 1.28 g of methanesulphonyl bromide was added at 0°C. The reaction mixture was kept under mixing for another 30 minutes and then heated at 80ºC for 16 hours. The mixture was cooled down to room temperature and the reaction was stopped by the addition of ca 10 mL of Milli-Q water. The mixture was neutralized with 0.1 N NaOH, concentrated under reduced pressure and poured in 200mL of acetone. The product was collected by filtration, washed with acetone, suspended in distilled water, and then dialysed against distilled water and freeze-dried Weight of the solid: 480 mg.

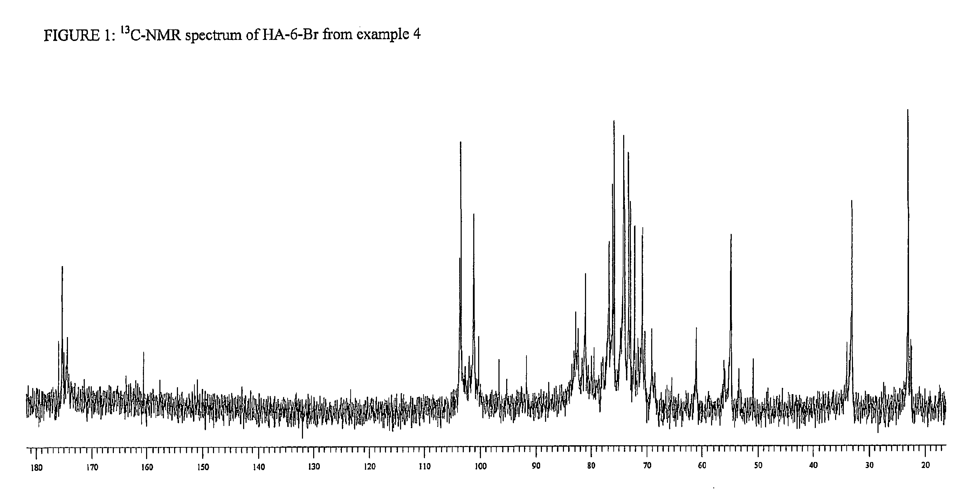

The Br content was determined from ¹³C-NMR (Figure 1) by integrating the peaks at 60 ppm (CH₂OH) and the peak at 33 ppm (CH₂Br) and resulted equal to 14% w/w; MW: 11800; PI:1.4 The ¹³CNMR spectrum shows the presence of many small peaks which could not be assigned either to HA or brominated HA, eg peaks at 51, 53, 91, 93 ppm. This NMR pattern of signals suggests the presence of side products deriving from the bromination reaction.

### EXAMPLE 5:6-Chloro-6-deoxy-hyaluronic acid (HA-6-Cl or HA-Cl)

To a suspension of 10 g of sodium salt of HA (MW 20,000) in 180 ml of dimethylformamide under stirring, mesylchloride (10 eq) was added dropwise in 30 minutes at -10°C under a N₂ blanket. The mixture was maintained for 30 minutes at this temperature and then allowed to reach 60°C over a period of 2 hr. The reaction was then maintained at 60°C for 16 hr with stirring. Then it was cooled to room temperature, poured in a mixture of ice and saturated NaHCO₃ and then treated with NaOH solution to pH 9. After one day at this pH the suspension became a solution that was neutralized with dilute HCl. The solution was then ultrafiltered, concentrated and was freeze-dried to recover a white solid (6g).

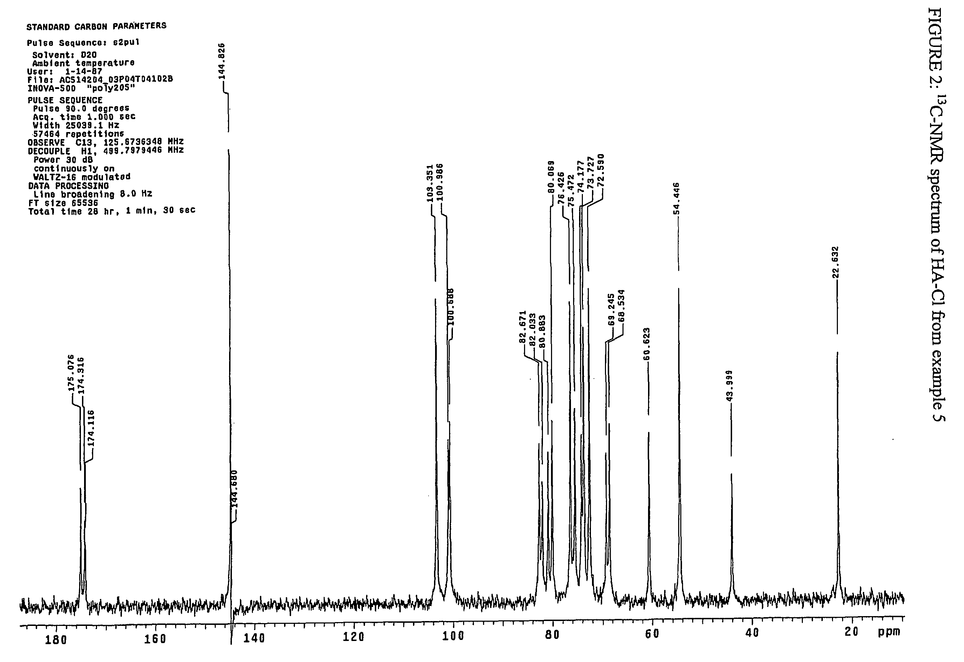

The ¹³C-NMR spectrum (Figure 2) of the product revealed the presence of the signal at 44 ppm (CH₂Cl) and integration of the peaks at 61 ppm and 44 ppm yielded a degree of chlorine substitution of 3.4 %w/w.

The comparison of spectrum in Figure 2 with spectrum in Figure 1 (HA-Br) shows that chlorination reaction yields a clean product. In fact, only 21 peaks are visible and could be assigned either at the underivatized HA repeating unit (14 Carbon atoms) or to the 6-Cl-(N-acetyl-D-glucosamine) sugar unit (7 Carbon atoms) with respect to the much higher number of peaks visible in HA-Br spectrum.

### EXAMPLE 6:HA-Cl

The reaction was carried out as in Example 5 with the difference that 15 eq of mesylchloride were used. 4.4 g were recovered.

The ¹³C-NMR spectrum was similar to compound from Example 5 revealing a degree of chlorine substitution of 4.3% w/w.

### EXAMPLE 7: HA-Cl

The reaction was carried out as described in Example 5 with the difference that 50 g of sodium salt of HA were used. 36 g of white solid were recovered. The ¹³C-NMR data were similar to those of the compound prepared in Example 5; the chlorine content was 3.4 % w/w (¹³C-NMR).

### EXAMPLE 8: HA-Cl

The reaction was carried out as described in Example 5 with the difference that the reaction mixture was maintained at 60 °C for 20.5 hours. 36 g of white solid were recovered.

The ¹³C-NMR data were similar to those of the compound prepared in Example 5; the chlorine content was 4.9% w/w (¹³C-NMR).

### EXAMPLE 9: HA-Cl

The reaction was carried out as described in Example 5 with the difference that the reaction mixture was maintained at 60 °C for 24.5 hours. 36 g of white solid were recovered.

The ¹³C-NMR data were similar to those of the compound prepared in Example 5; the chlorine content was 5.8% w/w (¹³C-NMR).

### EXAMPLE 10: HA-Cl

To a solution of HA TBA (2g, Mw 70,000) in 40 ml of dry dimethylformamide under stirring, mesylchloride (5 eq) was added dropwise in 30 minutes time at -10°C under N₂ flow. The mixture was maintained for 30 minutes at -10°C and 1 hour at room temperature and then heated at 60°C for 16 hr. The reaction was then brought to room temperature, 10% TBAOH solution was added to pH 9-10, and this pH was maintained for 4 days until the suspension became a brown solution. It was purified by ultrafiltration using a membrane with molecular weight cut off of 10KD. The aqueous solution was then freeze-dried to give 1 g of the desired product.

The ¹³C-NMR data were similar to those of the compound prepared in Example 5; the chlorine content was 1.75% w/w (¹³C-NMR ).

### EXAMPLE 11: HA-Cl

The reaction was carried out as in example 10 with the difference that the reaction temperature was 50°C, the time was 18 hr and 8 equivalents of mesylchloride was used. 1.4 g of the desired product were recovered and the ¹³C-NMR data were similar to those of the compound prepared in Example 5; the chlorine content was 3.1% w/w (¹³C-NMR).

### EXAMPLE 12: HA-Cl

The reaction was carried out as in example 10, with the difference that 10 grams of HA were used, the reaction temperature was 55°C, the time was 6 hr and 10 equivalents of mesylchloride were used. 8.9 g of the desired product were recovered. The ¹³C-NMR data were similar to those of the compound prepared in Example 5; the chlorine content was 1.4% w/w (¹³C-NMR).

### EXAMPLE 13: HA-Cl

The reaction was carried out as in example 10, with the difference that 20 g of HA were used, the reaction temperature was 50°C, and 8 equivalents of mesylchloride were used. 16 g of the desired product were recovered and the ¹³C-NMR data were similar to those of the compound prepared in Example 5; the chlorine content was 2.6% w/w (¹³C-NMR).

### EXAMPLE 14: HA-Cl TBA salt

To a solution of HATBA (50g, Mw 70,000) in 1000 ml of dry DMF under stirring, mesylchloride (10 eq) was added dropwise in 1 hr time at -10°C under N₂ flow.

The mixture was maintained for 1 hour at room temperature and then heated at 60°C for 16 hr. The work-up was performed as in Example 10 to obtain 46.9 g.

The ¹³C-NMR data were similar to those of the compound prepared in Example 5; the chlorine content was 4.2% w/w (¹³C-NMR).

### EXAMPLE 15: HA-Cl

HA-CI TBA salt was prepared by means of a cation exchange column packed with Amberlite IR-120 resin. The column (72 cm x 4 cm) was provided with a thermostatable external jacket, 200 g of dried resin were conditioned with in 700 mL of 1 M hydrochloric acid for 1 hour under mild stirring, washed with distilled water and poured into the column. The column was rinsed with more distilled water. 200 mL of TBA-OH 40% were added to the column and maintained under re-circulation of TBAOH, at a flow rate of 30 mL/min, for 72 hours at a temperature of 40°C. Finally, the resin was rinsed with several litres of water, until a final pH of 8.5-9 was obtained. 5.65 g of HA-Cl Na were dissolved in 160 mL of milliQ water. The solution was then poured into the column, previously drained of water, and recirculated in the column, by means of a peristaltic pump, for 24 hours at a flow rate of 4 mUmin. The HA-Cl TBA was then recovered by rinsing the column to yield 7.7 g of HA-Cl TBA.

### EXAMPLE 16: 6-deoxy-6-O-methotrexyl-hyaluronic acid (HA-6-MTX or HA-MTX)

To a solution of 3.5 g of HA-Cl TBA salt from Example 12 in 175 ml of anhydrous DMSO under stirring and under nitrogen, a solution of 2eq (per repeating unit of HA backbone) of methotrexate (5.14 g) in DMSO (51 ml) was added; then 2eq (per repeating unit) of solid cesium carbonate (3.68 g) were added in portions. The resulting mixture was stirred and heated at 65°C for 40h. After this time the mixture was cooled to room temperature and poured into ice water. The pH was adjusted to 7 with dilute HCl solution and the mixture stirred at room temperature for 4 hours. It was then dialysed against 1 M NaCl (2 x 2.5L), the insoluble material was filtered through a sintered glass filter (class IV), and the solution was ultrafiltered against a 10 KDa membrane and then filtered through 1.2µm, 0.45µm and 0.22µm pore size filters. The yellow solution was concentrated in vacuum and freeze-dried to obtain a yellow fluffy solid (0.8 g).

The ¹³C-NMR spectrum confirmed the occurrence of the linkage in position 6 of D-glucosamine residue: the peak at 64 ppm is assigned at CH₂O-MTX and its intensity corresponds to the decrease of the peak at 44 ppm (CH₂Cl) compared to the parent chlorine derivative. The MTX content, was 4.2% w/w (HPLC); content of free MTX was below 0.5% w/w (HPLC); Water content: 10.5% w/w; MW: **63000;** Pl: 2.8. Residual chlorine: 0.8 % w/w.

### EXAMPLE 17: HA-MTX

The conjugate was prepared as in Example 16 with the difference that a solution of HA-Cl TBA salt (1 g, from example 10) in 50 ml anhydrous DMSO was treated with methotrexate (1.65 g) and cesium carbonate (1.13 g) at 70°C for 40 hour, to afford, after the work up as described in Example 12, a yellow solid (0.7 g).

The ¹H-NMR DOESY and ¹³C-NMR data were similar to those of the compound prepared in Example 16 confirming the presence of MTX covalently bound on C-6 of HA. The MTX content was 10.0% w/w (HPLC); free MTX was 0.17%w/w (HPLC); Water content was 10% w/w, MW: **94000;** PI: 5.2.

### EXAMPLE 18: HA-MTX

The conjugate was prepared as in Example 16 with the difference that 1.52 g of HA-Cl-TBA salt from Example 11 in DMSO (75 ml) was treated with 2.4g MTX and 1.63g cesium carbonate at 70°C for 40 h, to give, after the usual work up, 450 mg of a yellow solid.

The structure was confirmed by ¹H-NMR DOESY and ¹³C-NMR. MTX content was 16% w/w (HPLC); free MTX was <0.5% (HPLC); Water content: 12% w/w, MW: **63000**, PI: 3.2.

### EXAMPLE 19: HA-MTX

The conjugate was prepared as in Example 16 with the difference that a solution of HA-Cl TBA salt (20 g, from Example 14) in DMSO (1.25 L) is treated with MTX (29.3g) and cesium carbonate (21 g) at 80°C for 40 h, giving 5.6 g of a yellow solid.

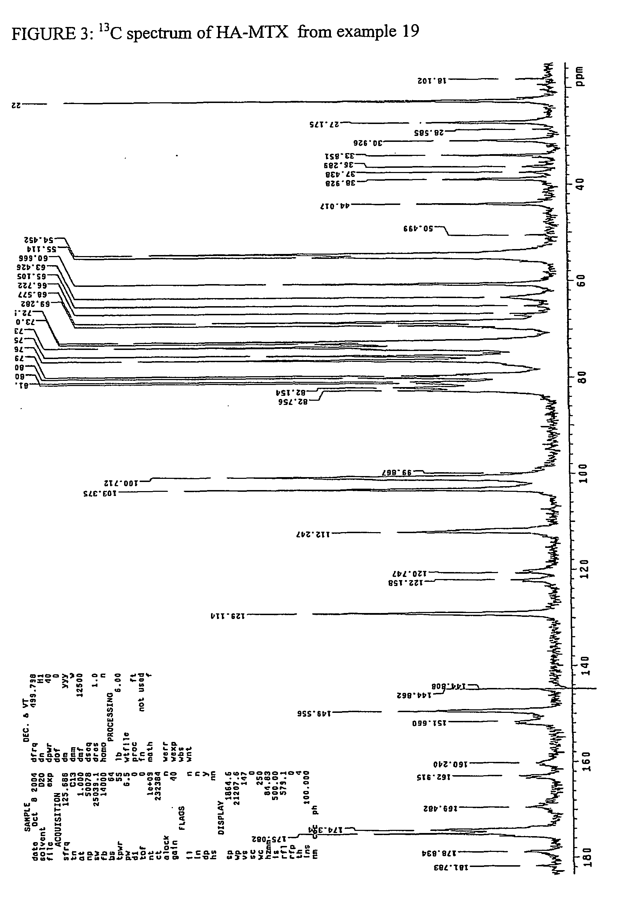

The structure of the conjugate was supported by NMR. ¹³C-NMR spectrum (Figure 3) confirmed the occurrence of the linkage in position 6 of N-acetyl-D-glucosamine: the peak at 64 ppm is assigned at CH₂O-MTX and its intensity corresponds to the decrease of the peak at 44 ppm (CH₂Cl) compared to the parent chlorine derivative. The MTX content was 18.8% w/w (HPLC); free MTX was 0.1% w/w, water content: 8.2% w/w; MW: **11000;** Pl: 1.4; residual chlorine content: 1.76% w/w (NMR).

### EXAMPLE 20: HA-MTX

To a solution of 5 g of HA-Cl TBA salt from example 5 in 150 ml of anhydrous DMSO under stirring and under nitrogen, a solution of 9 g of MTX in 100 ml of DMSO was added; then 6.43 g of solid caesium carbonate were added in portions. The resulting mixture was stirred and heated at 70°C for 40h. After this time the mixture was cooled at room temperature and poured into 200 ml ice water. The pH was decreased to 4.75 with HCl 1N and then adjusted to 7 with saturated NaHCO₃ solution (final volume of 500 ml). It was then dialysed against 1 M NaCl (2x2.5L), the insoluble material was filtered through sintered glass filters (class II, III and then IV), and the solution was ultrafiltered against a 10 KDa membrane and then filtered through 1.2 micron, 0.45 micron and 0.22 micron pore size filters. The yellow solution was concentrated in vacuum and freeze-dried to obtain a yellow fluffy solid (0.8 g).
¹H NMR DOSY and ¹³C-NMR data confirmed the linkage of MTX on position 6 of the D-glucosamine residue. The MTX content was 20% w/w (HPLC); free MTX was below 0.5% w/w (HPLC); Water content: 11.7% w/w. MW: 39000; PI: 3.3.

### EXAMPLE 21: 6-O-acetyl-6-O-methotrexyl-hyaluronic acid (HA-6-MTX-6-Ac or HA-MTX-Ac)

HA-Cl was obtained as described in Example 10 but starting from 50 g of HA TBA in 1 L of DMF, 8 eq of mesylchloride, 50 °C. 36 g of HA-Cl were obtained with a chlorine content of 2% w/w. 30 grams of said HA-Cl were reacted with MTX as described in Example 19. The resulting 15 g of HA-MTX derivative contained 7.5% w/w of MTX and 1.25 % w/w of residual chlorine groups. A suspension of 1g of HA-MTX-Cl Na salt in 100 ml anhydrous DMSO was heated at 60°C for 1 h. The homogeneous suspension was treated with 10 equivalents of anhydrous solid caesium acetate (CsAcO) at 100°C for 24 h under stirring. The mixture, after cooling at room temperature, was purified by ultrafiltration, and then freeze-dried yielding 0.63g of HA-MTX-Ac Na.

MTX content was 5% w/w (HPLC) Mw: 31000, Pl 2.2, water content 17% w/w. Acetate content was estimated by the integration of peak at 20.8 ppm corresponding to the methyl group of acetate and the peak at 23 ppm corresponding the CH₃ of acetamido group of HA and resulted equal to 1% w/w. The structure of HA-MTX-Ac was supported by the ¹³C-NMR spectra: the disappearance of the signal at 44 ppm confirms the complete displacement of chlorine groups and the presence of the signals at 63.8 ppm (CH₂-OAc) and at 20.8 ppm (CH₃ of the acetate group) confirmed the presence of acetate groups in position 6 of glucosamine.

### EXAMPLE 22: HA-MTX-Ac

A suspension of 2 g of the HA-MTX Na from Example 20 in 200 ml of anhydrous DMSO was treated with 10 equivalents of anhydrous solid caesium acetate (CsAcO) at 100°C for 19 h, under stirring. The reaction was worked up as described in Example 21 to give 1.46 g of HA-MTX-Ac Na salt.

MTX content: 13% w/w (HPLC). Mw = 27600, Pl 2.9, water content: 13 %w/w. Acetate content was 0.3% w/w. (determined as for Example 21). The ¹³C-NMR spectrum is similar to that of the compound prepared in Example 21.

### EXAMPLE 23: HA-MTX-Ac

A suspension of 2.5 g of the HA-MTX Na from Example 19 in 250 ml of anhydrous DMSO was treated with 10 equivalents of anhydrous solid caesium acetate (CsAcO) at 100°C for 19 h, under stirring. The reaction was worked up as described in Example 21 to give 1.6 g of HA-MTX-Ac Na salt with MTX content 11 % w/w (HPLC). Water content: 12.6 %w/w. Acetate content was 1.3% w/w. (determined as for Example 21).

The ¹³C-NMR data is similar to those of the compound prepared in Example 21.

### EXAMPLE 24: 6-O-Butyryl-6-O-methotrexyl-hyaluronic acid (HA-6-MTX-6-But or HA-MTX-But)

21 g of HA-Cl from Example 14 were reacted with 2.67 eq of MTX and 2.67 eq of Cs₂CO₃ at 80°C for 40 hours. 200 mg of the resulting HA-MTX-Cl with a MTX content of 30%w/w, were suspended in 20 ml of DMSO in the presence of 5 eq of cesium butyrate (CsBut) at 80°C for 21 hours. The reaction was worked up as described in Example 22 to give 0.195 g of HA-MTX-But MTX content was 20% w/w (HPLC). Mw= 13000, Pl = 1.5, water content: 11.5% w/w. Butyrate content was determined by the integration of signals at 23 ppm (CH₃ of acetamido group) and 13.3 ppm (CH₃ of butyryl group) and resulted of 3.5 % w/w.

The structure of HA-MTX-But:Na is supported by its ¹³C-NMR spectra. The disappearance of the signal at 44 ppm confirms the complete displacement of chlorine groups and the presence of the signals at 63.8 ppm (CH₂-OBut) and at 13.3 ppm (CH₃ of butyrate group) confirmed the presence of butyrate groups in position 6 of glucosamine.

### EXAMPLE 25: HA-MTX-But

10 g of HA-Cl from Example 8 were reacted with 2 eq of MTX and 2 eq of Cs₂CO₃ at 80°C for 40 hours. 1.7 g of the resulting HA-MTX-Cl with a MTX content of 24%w/w, were suspended in 150 ml of dry DMSO in the presence of 2.5 eq of CsBut at 85°C for 21 hours. The reaction was worked up as described in Example 22 to give 1.2 g of HA-MTX-But. MTX content was 12.7% w/w (HPLC). Water content: 11.5% w/w. Butyrate content was determined by the integration of signals at 23 ppm (CH₃ of acetamido group) and 13.3 ppm (CH₃ of butyryl group) and resulted equal to 1.7 %w/w.

### EXAMPLE 26: in-vitro antiproliferative activity

Antiproliferative activity of the conjugates was determined on 2 lines of human mammary carcinoma (MCF-7, MDA-MB-231), and on 2 lines of human ovary carcinoma (SK-OV-3, IGR-OV1) with different Reduced Folate Carrier (RFC) expression, and on a non-tumour line originating from healthy mammary ductal epithelium (HBL-100 line). Cells were incubated in 96-well plates for 5 days in complete medium (consisting of DMEM/F12, with10% FBS, foetal bovine serum, 1% L-glutamine 100x (200 mM) and 1% penicillin-streptomycin (Euroclone) for MCF-7, IGROV-1 and MDA-MB-231; RPMI-1640 with 10% FBS, foetal bovine serum, 1% L-glutamine 100x (200 mM) and 1% penicillin-streptomycin (Euroclone) for SK-OV-3; McCoy's with 10% FBS, foetal bovine serum, 1% L-glutamine 100x (200 mM) and 1% penicillin-streptomycin (Sigma) for HBL-100 at 37°C and controlled atmosphere (5% CO₂) with MTX-Na or with conjugate; the conjugates were tested at doses equimolar with those of MTX-Na, in the range 1-30 nM .

Cytotoxicity was determined on day 6 (after 5 days treatments) by the MTT test, by measuring cell viability as the cell metabolic capacity to transform the tetrazolium salt of MTT in the blue formazan, by mitochondrial dehydrogenases; the blue colour is read at 570 nm with a spectrophotometer. The following conjugates were tested.

| Example | Conjugate | Type of conjugate |
|---|---|---|
| Ex 21 | HA-MTX-Ac | MTX 5% Ac 1% |
| Ex 22 | HA-MTX-Ac | MTX 13% Ac 0.3% |
| Ex 24 | HA-MTX-But | MTX 20% But 3.5% |
| Ex 16 | HA-MTX | MTX 4.2% |
| Ex 17 | HA-MTX | MTX 10% |
| Ex 18 | HA-MTX | MTX 16% |

Antitumour effect of the conjugates of the invention on various tumour cells is reported in tables A and B; the effect of these conjugates have been compared to that of the HA-MTX of examples 16-18, which are the intermediate conjugates used for the preparation of the conjugates of the invention. The values of the concentration (IC₅₀, µM) of conjugate which is necessary to reduce the growth of breast carcinoma cells to 50% of the growth of the control are shown in Table A.

**Table A**

| compound | MDA-MB-231 |
|---|---|
| MXT | 1.80 |
| Ex 21 | 2.7 |
| Ex 24 | 2.18 |
| Ex 22 | 3.03 |
| Ex 17 | 12.0 |
| Ex 18 | 12.0 |
| Ex 16 | 18.8 |

The above data show that the conjugates between hyaluronic acid and methotrexate produce the same inhibition % as the non-conjugated methotrexate at a concentration 10 times higher. Moreover, the data show that, surprisingly, the further esterification of the conjugates on the primary hydroxyl group of hyaluronic acid results in a significant increase in the antiproliferative activity. In fact, the conjugates of Examples 21, 22 and 24 produce the same inhibition % as the non-conjugated methotrexate at a concentration 4-5 times lower than the conjugates of Examples 16-18.

Table B shows the values of the concentration (IC₅₀, µM) of the conjugates and of MTX necessary to reduce the cell growth of various tumours lines to 50% of the growth of the control.

**Table B**

| Cell line | MTX | Ex.21 | Ex 24 | Ex 22 |
|---|---|---|---|---|
| MCF-7 | 0.081 | 1.97 | 1,70 | 1,23 |
| IGR-OV1 | 0.14 | 2,74 | 2,16 | 1,98 |
| SK-OV-3 | 0.041 | 1,82 | 1,67 | 1,57 |
| HBL-100 | 0.50 | 2,05 | 2,11 | 2,19 |

These further data shows that the conjugates have significant antiproliferative activity on a wide range of tumour cell lines. In fact, IC₅₀ values are also very low and are comparable to the values obtained for the conjugates of the invention on the cell line of table A.

### EXAMPLE 27: in-vivo antiproliferative activity

BD2F1 mice weighing 18-20 g were implanted intraspleen (i.s) with 100.000 B16/F10 melanoma cells on Day 0, under sterile conditions and Zoletil® (70 mg/kg), anaesthesia, following surgical opening of peritoneum and spleen immobilisation. Tumour cells, suspended in diluted Matrigel® (150 µg/ml), were injected i.s. in a volume of 0.05 ml. The B16F10 melanoma cells were cultured in Modified Eagle Medium. Cells were maintained in a humidified 5% CO₂ atmosphere at 37°C. Animals were then randomly divided into 5 groups (each of 7-8 female), implanted i.s. with 100.000 B16/F10 melanoma cells on Day 0, were treated i.v. (on Days 3, 6 and 9) with HA-MTX at 2, 1 and 0.50 mg/kg/day or MTX-Na at 8.5, 6 and 3 mg/kg/day. Doses are referred to equivalents of MTX. The data originate from 4 separate experiments.

The evaluation of livers and counting of liver metastases under a low power stereo microscope equipped with a calibrated grid was performed on Day 21. Livers were then stored in formaldehyde for preparation for light microscopy observations.

The administration of HA-MTX of example 18, both after i.v. and i.p. administration, was effective in preventing the formation of liver metastasis

Results are reported in Table 10.

| **Treatment** | Spleen weight 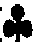 | Hepatic metastases | |
|---|---|---|---|
| *(**mg MTX*/*kg*/*die)* | **(mg)** | **Metastatic involvement** | **Animals free** 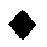 |
| ***Controls*** | 194 ±69 | +++++ 84%^; 32%° | 0/25 |
| ***MTX-Na (8.5) on days 3,6,9*** | 149 ±24 | + 13%^; 0%° | 8/16** |
| ***MTX-Na (6) on days 3,6,9*** | 159 ±7 | ++ 21%^; 0%° | 7/14** |
| ***MTX-Na (3) on days 3,6,9*** | 227 ±36 | +++ 38%^; 19%° | 6/16* |
| ***HA-MTX (ex18) (2) on days 3,6,9*** | 150 ±37 | - 0%^; 0%° | 14/14***^{§} |
| ***HA-MTX (ex18) (1) on days 3,6,9*** | 199 ±27 | ++ 31%^; 0%° | 9/16*** |
| ***HA-MTX (ex18) (0.5)* on *days 3,6,9*** | 155 ±16 | +++ 27%^; 13%° | 6/15** |

| | | | |
|---|---|---|---|
| ^ indicates the presence of mice with more than 3 visible hepatic tumour nodules; o indicates the presence of mice with metastases larger than 3 mm. 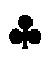 : Spleen weight on Day 21 (for comparison, the spleen of a healthy mouse of the same strain and body weight is 117±11 mg). 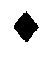 : Number of mice free of macroscopically detectable liver metastases over the total number of mice per group. Statistical difference versus controls: * p<0.01; ** p<0.001; *** p<0.0001 and §: is different from all other (Fisher's test). | | | |

The above data show that HA-MTX causes the complete disappearance of liver metastases of B16/F10 melanoma after i.v. treatment at a dose of 2 mg/kg/day (MTX equivalents) given on days 3,6,9 following tumour implantation. Moreover liver metastasis reduction at the lower dose of 1 and 0.5 mg/kg/day is comparable to that of 6 and 3 mg/kg/day MTX-Na respectively, and corresponds to 1/6 of the MTX-Na dose.

### EXAMPLE 28: Subacute toxicity

Groups of 3 or 7 CBA/Lac male mice were given a repeated i.p. injection of conjugates for 5 consecutive days. Doses were calculated in order to give the animals an amount of MTX of 1.5 and 3.0 mg/kg/day MTX. Treatment was performed with each dose dissolved in a volume not exceeding 250 µl/animal. Mortality was evaluated over a follow-up period of 21 days after the end of the treatment. On the same animals, body weight variation was measured between the day before treatment and 4-days after.

**Table C**

| Compound | Dose (mg/kg MTX) | Lethality N. deaths/N. total | Body weight variation (g) |
|---|---|---|---|
| Ex 19 | 3(mg/kg)*5die | 7/7 | -0.40 |
| Ex 19 | 1,5(mg/kg)*5die | 0/3 | -0.10 |
| Ex 23 | 3(mg/kg)*5die | 0/3 | +0.10 |
| Ex 23 | 1,5(mg/kg)*5die | 0/3 | +0.20 |
| Ex 25 | 3(mg/kg)*5die | 0/3 | +0.10 |
| Ex 25 | 1,5(mg/kg)*5die | 0/3 | +0.20 |

From the data of subacute toxicity it is clear that the conjugates that have been further esterified (Examples 23 and 25) are less toxic than the conjugates without further esterification (Ex 19).

## Claims

1. Conjugates of hyaluronic acid, either in the acid or in the salt form, wherein the primary hydroxyl groups of the N-acetyl-D-glucosamine unit of hyaluronic acid are esterified both with the carboxylic group of a compound A selected from:
(i): an aliphatic acid selected from the group consisting of acetic acid, butyric acid, propionic acid, retinoic acid, n-propylacetic acid, succinic acid, cyclohexanecarboxylic acid, cyclohexaneacetylic acid and cyclopropanecarboxylic acid;
(ii) an aminoacid selected from the group consisting of alanine, valine, leucine, isoleucine, methionine, glycine, serine, cysteine, asparagine, glutamine, aspartic acid, glutamic acid, proline, histidine phenylalanine, triptophane and tyrosine;
(iii) an arylaliphatic acid selected from the group consisting of phenylacetic acid, phenoxyacetic acid, naphtylacetic acid, 2-(4-isobutylphenyl)propionic acid, 2-(6-methoxy-2-naphtyl) propionic acid and cinnamic acid;
(iv) substituted or unsubstituted benzoic acid.
and with 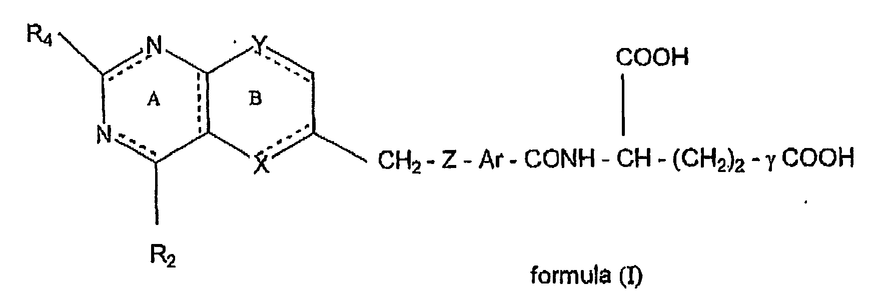 wherein:
R₂ and R₄, independently from one another represent: -NH₂, -OH, -OCH₃, C₁-C₅ alkyl, =O;
X and Y represent: -C(R₅)=, -CH(R₅)-, -NH-, -N=, wherein R₅ represents: -H, C₁-C₅ alkyl;
Z represents: -CH(R₁₀)-, -N(R₁₀)-, -O-;
R₁₀ represents: -H, C₁-C₅ alkyl, C₁-C₅ alkenyl, C₁-C₅ alkynyl, 5-6 membered heterocyclic ring with 1-3 heteroatoms selected from the group consisting of nitrogen, sulphur and oxygen;
Ar represents: 1,4-phenyl group, 1,4-phenyl group condensed with one or more 6-6 membered aromatic rings, 1,4-phenyl group condensed with one or more 5-6 membered heterocycles, wherein said groups are possibly substituted with R₂;
rings A and B, indipendently from one another, may be aromatic or non-aromatic.

2. Conjugates as claimed in claim 1 wherein said compound of formula (I) is methotrexate.

3. Conjugates as claimed in claims 1 or 2 wherein the total amount of compound of formula (I) and of compound A is comprised between 0.1% and 60% w/w with respect to the total weight of the conjugate.

4. Conjugates as claimed in claims 1 to 3 wherein said compound of formula (I) is present in amount ranging from 1% to 40% w/w and said compound A in an amount ranging from 0.1 to 30% w/w, with respect to the total weight of the conjugate.

5. Conjugate as claimed in claim 4 wherein said compound A is acetic acid and it is present in an amount ranging from 0.1% to 10% w/w with respect to the total weight of the conjugate.

6. Conjugates as claimed in claims 1 to 5 wherein said aliphatic acid is substituted with a substituent group selected from the group consisting of linear or branched C₁-C₅ alkyls, halogens, nitro groups, cyano groups, hydroxyl groups, amino groups, methoxyl groups, carbonyl groups, thiol groups and carboxyl groups.

7. Conjugates as claimed in claims 6 wherein said substituted benzoic acid is selected from the group consisting of halobenzoic acid, alkylbenzoic acid, nitrobenzoic acid and 2-acetoxybenzoic acid.

8. Conjugates as claimed in claims 1 to 4 wherein said compound A is selected from the group consisting of acetic acid, butyric acid, alanine, glycine and peptides containing alanine and/or glycine.

9. Conjugates as claimed in claims 1 to 8 salified with a metal selected from the group consisting of akaline metals, earth-alkaline metals and transition metals.

10. Conjugates as claimed in claim 9 salified with a metal selected from the group consisting of Na⁺, K⁺, Ca⁺⁺, Mg⁺⁺, Cu⁺⁺ Zn⁺⁺, Ag⁺⁺, Au⁺⁺ and Co⁺⁺.

11. Conjugates as claimed in claims 1 to 10 wherein the secondary hydroxyl groups of the hyaluronic acid are derivatised to form a group selected from the group consisting of -OR, -OCOR, -SO₂H, -OPO₃H₂, -O-CO-(CH₂)ₙ-COOH and -O-(CH₂)ₙ-OCOR, wherein n is 1-4 and R is C₁-C₁₀ alkyl.

12. Conjugates as claimed in claims 1 to 10 wherein the secondary hydroxyl groups of the hyaluronic acid derivatised to form a group selected from -NH₂ and - NHCOCH₃.

13. Use of conjugates as claimed in claims 1 to 12 in the manufacture of a medicament for the treatment of pathologies **characterised by** cell hyperproliferation.

14. Use as claimed in claim 13 wherein said pathologies are selected from the group consisting of tumours, skin disorders, psoriasis, inflammatory pathologies and rheumatoid arthritis.

15. Pharmaceutical compositions containing conjugates as claimed in claims 1 to 12 in admixture with pharmaceutically acceptable excipients and/or diluents.

16. Process for the preparation of conjugates as claimed in claims 1 to12.

17. Process as claimed in claim 16 which comprises the following steps to be carried out in the order indicated:
(a) chlorination of the primary hydroxyl groups of the *N*-acetyl-D-glucosamine units of the hyaluronic acid either in free or salt form;
(b) formation of ester linkages between the chlorinated hyaluronic acid and the carboxyl groups of the compound of formula (I) by displacement of chlorine atoms,
(c) formation of ester linkage between the product of step (b) and compound A by displacement of the residual chlorine atoms with a suitable acyl nucleophile of compound A.

18. Process as claimed in claim 17 wherein the reagent used for the chlorination is methanesulphonyl chloride in *N,N*-dimethylformamide.

19. Process for obtaining conjugates between hyaluronic acid and a compound of formula (I): 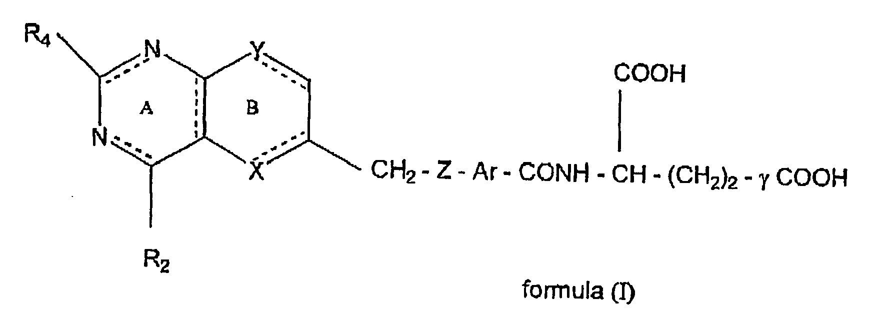 wherein:
R₂ and R₄ represent: -NH₂, -OH, -OCH₃, C₁-C₅ alkyl, =O;
X and Y represent: -C(R₅)=, -CH(R₅)-, -NH-, -N=, wherein R₅ represents: -H, C₁-C₅ alkyl;
Z represents: -CH(R₁₀)-, -N(R₁₀)-, -O-, wherein R₁₀ represents: -H, C₁-C₅ alkyl, C₁-C₅ alkenyl, C₁-C₅ alkynyl, a 5-6 membered heterocyclic ring with 1-3 heteroatoms selected in the group consisting of nitrogen, sulphur and oxygen;
Ar represents: 1,4-phenyl group, 1,4-phenyl group condensed with one or more 5-6 membered aromatic rings, 1,4-phenyl group condensed with one or more 5-6 membered heterocycles, wherein said groups are possibly substituted with R₂;
rings A and B are aromatic or non-aromatic;
**characterised in that** they contain less than 3% w/w of residual chlorine chemically linked to the polymeric chain of hyaluronic acid,
said process comprising the following steps, carried out in the order indicated:
(a) chlorination of the primary hydroxyl groups of the N-acetyl-D-glucosamine units of the hyaluronic acid either in free or salt form; and
(b) formation of ester linkages between the chlorinated hyaluronic acid and the carboxyl groups of the compound of formula (I) by displacement of the chlorine atoms.

20. Process for obtaining conjugates as claimed in claim 19 comprising the following steps, carried out in the order indicated:
a) chlorination of the primary hydroxyl groups of the N-acetyl-D-glucosamine units of the hyaluronic acid either in free or salt form;
(b) formation of ester linkages between the chlorinated hyaluronic acid and the carboxyl groups of the compound of formula (I) by displacement of chlorine atoms;
(c) displacement of the residual chlorine atoms of the product of step (b) by a suitable acyl nucleophile and formation of ester linkages; and
d) selective deacylation of the conjugates obtained from step (c).

## Patentansprüche

1. Konjugate von Hyaluronsäure, entweder in der Säure- oder in der Salz-Form, worin die primären Hydroxyl-Gruppen der N-Acetyl-D-Glucosamin-Einheit von Hyaluronsäure verestert sind sowohl mit der Carboxyl-Gruppe einer Verbindung A, die ausgewählt ist aus:
(i) einer aliphatischen Säure, die ausgewählt ist aus der Gruppe, die besteht aus Essigsäure, Buttersäure, Propionsäure, Retinsäure, n-Propylessigsäure, Bernsteinsäure, Cyclohexancarbonsäure, Cyclohexanacetylsäure und Cyclopropancarbonsäure;
(ii) einer Aminosäure, die ausgewählt ist aus der Gruppe, die besteht aus Alanin, Valin, Leucin, Isoleucin, Methionin, Glycin, Serin, Cystein, Asparagin, Glutamin, Asparaginsäure, Glutaminsäure, Prolin, Histidin, Phenylalanin, Tryptophan und Tyrosin;
(iii) einer arylaliphatischen Säure, die ausgewählt ist aus der Gruppe, die besteht aus Phenylessigsäure, Phenoxyessigsäure, Naphthylessigsäure, 2-(4-Isobutylphenyl-)propionsäure, 2-(6-Methoxy-2-naphthyl-)propionsäure und Zimtsäure;
(iv) substituierter oder unsubstituierter Benzoesäure;
als auch mit 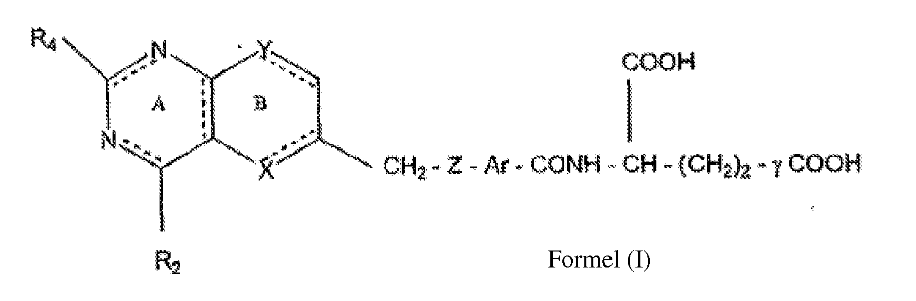 worin
- R₂ und R₄ unabhängig voneinander -NH₂, -OH, -OCH₃, C₁- bis C₅-Alkyl, =O wiedergeben;
- X und Y -C(R₅)=, -CH(R₅)-, -NH-, -N= wiedergeben, worin R₅ -H, C₁- bis C₅-Alkyl wiedergibt;
- Z -CH(R₁₀)-, -N(R₁₀)-, -O- wiedergibt;
- R₁₀ -H, C₁- bis C₅-Alkyl, C₁- bis C₅-Alkenyl, C₁- bis C₅-Alkinyl, einen 5- bis 6-gliedrigen heterocyclischen Ring mit 1 bis 3 Heteroatomen, die ausgewählt sind aus der Gruppe, die besteht aus Stickstoff, Schwefel und Sauerstoff, wiedergibt;
- Ar eine 1-4-Phenyl-Gruppe, eine 1,4-Phenyl-Gruppe, die mit einem oder mehreren 5- bis 6-gliedrigen aromatischen Ring(en) kondensiert ist, eine 1,4-Phenyl-Gruppe, die mit einem oder mehreren 5- bis 6-gliedrigen Heterocyclus/Heterocyclen kondensiert ist, worin diese Gruppen gegebenenfalls substituiert sind mit R₂, wiedergibt;
- die Ringe A und B unabhängig voneinander aromatisch oder nicht-aromatisch sein können.

2. Konjugate wie in Anspruch 1 beansprucht, worin die Verbindung der Formel (I) Methotrexat ist.

3. Konjugate wie in den Ansprüche 1 oder 2 beansprucht, worin die Gesamtmenge der Verbindung der Formel (I) und der Verbindung A zwischen 0,1 % und 60 % (w/w) liegt, bezogen auf das Gesamtgewicht des Konjugats.

4. Konjugate wie in den Ansprüchen 1 bis 3 beansprucht, worin die Verbindung der Formel (I) in einer Menge in einem Bereich von 1 % bis 40 % (w/w) zugegen ist, und die Verbindung A in einer Menge in einem Bereich von 0,1 bis 30 % (w/w) zugegen ist, bezogen auf das Gesamtsgewicht des Konjugats.

5. Konjugat wie in Anspruch 4 beansprucht, worin die Verbindung A Essigsäure ist und in einer Menge in einem Bereich von 0,1 % bis 10 % (w/w) zugegen ist, bezogen auf das Gesamtgewicht des Konjugats.

6. Konjugate wie in den Ansprüchen 1 bis 5 beansprucht, worin die aliphatische Säure substituiert ist mit einer Substituenten-Gruppe, die ausgewählt ist aus der Gruppe, die besteht aus linearen oder verzweigten C₁- bis C₅-Alkylen, Halogenen, Nitro-Gruppen, Cyano-Gruppen, Hydroxyl-Gruppen, Amino-Gruppen, Methoxyl-Gruppen, Carbonyl-Gruppen, Thiol-Gruppen und Carboxyl-Gruppen.

7. Konjugate wie in Anspruch 6 beansprucht, worin die substituierte Benzoesäure ausgewählt ist aus der Gruppe, die besteht aus Halogenbenzoesäure, Alkylbenzoesäure, Nitrobenzoesäure und 2-Acetoxybenzoesäure.

8. Konjugate wie in den Ansprüchen 1 bis 4 beansprucht, worin die Verbindung A ausgewählt ist aus der Gruppe, die besteht aus Essigsäure, Buttersäure, Alanin, Glycin und Peptiden, die Alanin und/oder Glycin enthalten.

9. Konjugate wie in den Ansprüchen 1 bis 8 beansprucht, die mit einem Metall, welches ausgewählt ist aus der Gruppe, die besteht aus Alkalimetallen, Erdalkalimetallen und Übergangsmetallen, in ein Salz überführt wurden.

10. Konjugate wie in Anspruch 9 beansprucht, die mit einem Metall, welches ausgewählt ist aus der Gruppe, die besteht aus Na⁺, K⁺, Ca⁺⁺, Mg⁺⁺, Cu⁺⁺, Zn⁺⁺ Ag⁺⁺, Au⁺⁺ und Co⁺⁺, in ein Salz überführt wurden.

11. Konjugate wie in den Ansprüchen 1 bis 10 beansprucht, worin die sekundären Hydroxyl-Gruppen der Hyaluronsäure derivatisiert sind, um eine Gruppe zu bilden, die ausgewählt ist aus der Gruppe, die besteht aus -OR, -OCOR, -SO₂H, -OPO₃H₂, -O-CO-(CH₂)ₙ-COOH und -O-(CH₂)ₙ-OCOR, worin n für 1 bis 4 steht und R für C₁- bis C₁₀-Alkyl steht.

12. Konjugate wie in den Ansprüche 1 bis 10 beansprucht, worin die sekundären Hydroxyl-Gruppen der Hyaluronsäure derivatisiert sind, um eine Gruppe zu bilden, die ausgewählt ist aus -NH₂ und -NHCOCH₃.

13. Verwendung der Konjugate wie in den Ansprüchen 1 bis 12 beansprucht zur Herstellung eines Medikaments zur Behandlung von Erkrankungen, die **gekennzeichnet sind durch** Zell-Hyperproliferation.

14. Verwendung wie in Anspruch 13 beansprucht, worin die Erkrankungen ausgewählt sind aus der Gruppe, die besteht aus Tumoren, Hauterkrankungen, Psoriasis, entzündlichen Erkrankungen und rheumatoider Arthritis.

15. Pharmazeutische Zusammensetzungen, die Konjugate enthalten, wie in den Ansprüchen 1 bis 12 beansprucht, unter Beimischung von pharmazeutisch annehmbaren Hilfsstoffen und/oder Verdünnungsmitteln.

16. Verfahren zur Herstellung der Konjugate, wie in den Ansprüchen 1 bis 12 beansprucht.

17. Verfahren wie in Anspruch 16 beansprucht, welches die folgenden Schritte umfasst, die in der angegebenen Reihenfolge durchgeführt werden sollen:
(a) Chlorieren der primären Hydroxyl-Gruppen der N-Acetyl-D-Glucosamin-Einheiten der Hyaluronsäure, entweder in der freien oder der Salz-Form;
(b) Bildung von Ester-Bindungen zwischen der chlorierten Hyaluronsäure und den Carboxyl-Gruppen der Verbindung der Formel (I) durch Entfernen der Chloratome,
(c) Bildung von Ester-Bindungen zwischen dem Produkt von Schritt (b) und der Verbindung A durch Entfernen der restlichen Chloratome mit einem geeigneten Acyl-Nucleophil von Verbindung A.

18. Verfahren wie in Anspruch 17 beansprucht, worin das für die Chlorierung verwendete Reagenz Methansulphonylchlorid in N,N-Dimethylformamid ist.

19. Verfahren zum Erhalt von Konjugaten zwischen Hyaluronsäure und einer Verbindung der Formel (I) 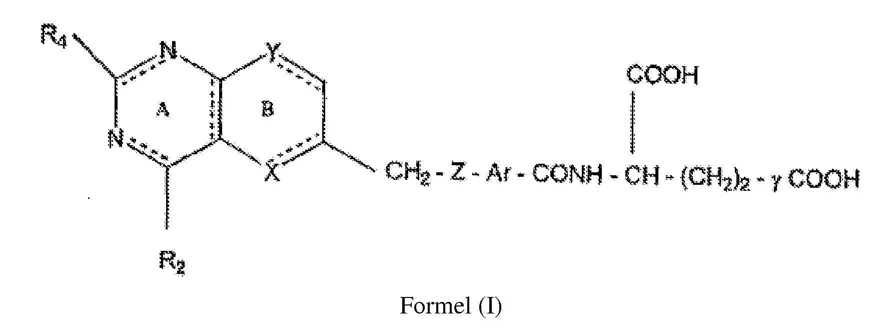 worin
- R₂ und R₄ stehen für -NH₂, -OH, -OCH₃, C₁- bis C₅-Alkyl, =O;
- X und Y -C(R₅)=, -CH(R₅)-, -NH-, -N= wiedergeben, worin R₅ -H, C₁- bis C₅-Alkyl wiedergibt;
- Z -CH(R₁₀)-, -N(R₁₀)-, -O- wiedergibt; worin R₁₀ -H, C₁- bis C₅-Alkyl, C₁- bis C₅-Alkenyl, C₁- bis C₅-Alkinyl, einen 5- bis 6-gliedrigen heterocyclischen Ring mit 1 bis 3 Heteroatomen, die ausgewählt sind aus der Gruppe, die besteht aus Stickstoff, Schwefel und Sauerstoff, wiedergibt;
- Ar eine 1-4-Phenyl-Gruppe, eine 1,4-Phenyl-Gruppe, die mit einem oder mehreren 5- bis 6-gliedrigen aromatischen Ring(en) kondensiert ist, eine 1,4-Phenyl-Gruppe, die mit einem oder mehreren 5- bis 6-gliedrigen Heterocyclus/Heterocyclen kondensiert ist, worin diese Gruppen gegebenenfalls substituiert sind mit R₂, wiedergibt;
- die Ringe A und B aromatisch oder nicht-aromatisch sind;
**dadurch gekennzeichnet, dass** sie weniger als 3 % (w/w) an restlichem Chlor enthalten, das chemisch an die Polymerkette der Hyaluronsäure gebunden ist;
worin das Verfahren die folgenden Schritte umfasst, die in der angegebenen Reihenfolge durchgeführt werden:
(a) Chlorieren der primären Hydroxyl-Gruppen der N-Acetyl-D-Glucosamin-Einheiten der Hyaluronsäure, entweder in der freien oder der Salz-Form; und
(b) Bildung von Ester-Bindungen zwischen der chlorierten Hyaluronsäure und den Carboxyl-Gruppen der Verbindung der Formel (I) durch Entfernen der Chloratome.

20. Verfahren zum Erhalt von Konjugaten, wie in Anspruch 19 beansprucht, das die folgenden Schritte umfasst, die in der angegebenen Reihenfolge durchgeführt werden:
(a) Chlorieren der primären Hydroxyl-Gruppen der N-Acetyl-D-glucosamin-Einheiten der Hyaluronsäure, entweder in der freien oder der Salz-Form;
(b) Bildung von Ester-Bindungen zwischen der chlorierten Hyaluronsäure und den Carboxyl-Gruppen der Verbindung der Formel (I) durch Entfernen der Chloratome,
(c) Entfernen der restlichen Chloratome von dem Produkt aus Schritt (b) durch ein geeignetes Acyl-Nucleophil und Bildung von Ester-Bindungen; und
(d) selektive Deacylierung der Konjugate, die in Schritt (c) erhalten wurden.

## Revendications

1. Conjugués d'acide hyaluronique, sous forme d'acide ou de sel, dans lesquels les groupes hydroxyle primaires de l'unité N-acétyle-D-glucosamine de l'acide hyaluronique sont estérifiés à la fois avec le groupe carboxylique d'un composé A choisi parmi :
(i) un acide aliphatique choisi parmi le groupe constitué d'acide acétique, d'acide butyrique, d'acide propionique, d'acide rétinoïque, d'acide n-propylacétique, d'acide succinique, d'acide cyclohexanecarboxylique, d'acide cyclohexaneacétylique et d'acide cyclopropanecarboxylique,
(ii) un aminoacide choisi parmi le groupe constitué d'alaline, de valine, de leucine, d'isoleucine, de méthionine, de glycine, de sérine, de cystéine, d'asparagine, de glutamine, d'acide aspartique, d'acide glutamique, de proline, d'histidine, de phénylalanine, de triptophane et de tyrosine,
(iii) un acide arylaliphatique choisi parmi le groupe constitué d'acide phénylacétique, d'acide phénoxyacétique, d'acide naphtylacétique, d'acide 2-(4-isobutylphényl)propionique, d'acide 2-(6-méthoxy-2-naphtyl)propionique et d'acide cinnamique,
(iv) d'acide benzoïque substitué ou non substitué,
et avec 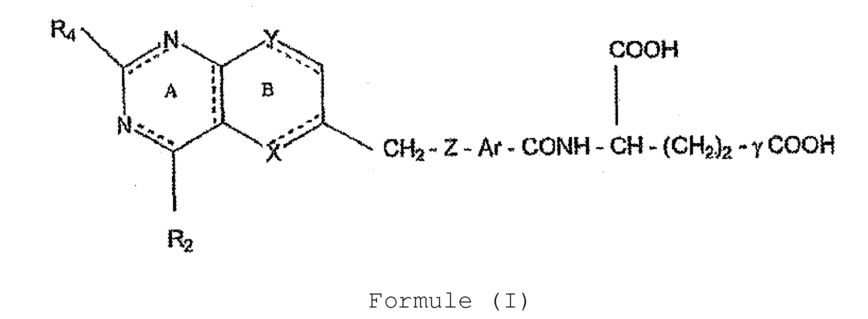 dans laquelle :
R₂ et R₄, indépendamment l'un de l'autre, représentent : -NH₂, -OH, -OCH₃, alkyle en C₁-C₅,=O,
X et Y représentent -C(R₅)=, -CH(R₅)-, -NH-, -N=, R₅ représentant : H, alkyle en C₁-C₅,
Z représente -CH(R₁₀)-, -N(R₁₀)-, -O-,
R₁₀ représente : -H, un alkyle en C₁-C₅, un alcényle en C₁-C₅, un alcynyle en C₁-C₅, un noyau hétérocyclique à 5 ou 6 chaînons avec 1 à 3 hétéroatomes choisis parmi le groupe constitué de l'azote, du soufre et de l'oxygène,
Ar représente : un groupe 1,4-phényle, un groupe 1,4-phényle condensé avec un ou plusieurs noyaux aromatiques à 5 ou 6 chaînons, un groupe 1,4-phényle condensé avec un ou plusieurs hétérocycles à 5 ou 6 chaînons, lesdits groupes étant éventuellement substitués par R₂,
les noyaux A et B, indépendamment l'un de l'autre, peuvent être aromatiques ou non aromatiques.

2. Conjugués selon la revendication 1, dans lesquels ledit composé de formule (I) est du méthotrexate.

3. Conjugués selon les revendications 1 ou 2, dans lesquels la quantité totale de composé de formule (I) et de composé A est comprise entre 0,1 % et 60 % p/p par rapport au poids total du conjugué.

4. Conjugués selon les revendications 1 à 3, dans lesquels ledit composé de formule (I) est présent dans une quantité variant de 1 % à 40 % p/p et ledit composé A dans une quantité variant de 0,1 à 30 % p/p par rapport au poids total du conjugué.

5. Conjugués selon la revendication 4, dans lesquels ledit composé A est l'acide acétique et est présent dans une quantité variant de 0,1 % à 10 % p/p par rapport au poids total du conjugué.

6. Conjugués selon les revendications 1 à 5, dans lesquels ledit acide aliphatique est substitué par un groupe substituant choisi parmi le groupe constitué d'alkyles en C₁-C₅ linéaires ou ramifiés, d'halogènes, de groupes nitro, de groupes cyano, de groupes hydroxyle, de groupes amino, de groupes méthoxyle, de groupes carbonyle, de groupes thiol et de groupes carboxyle.

7. Conjugués selon la revendication 6, dans lesquels ledit acide benzoïque substitué est choisi parmi le groupe constitué d'acide halobenzoïque, d'acide alkylbenzoïque, d'acide nitrobenzoïque et d'acide 2-acétoxybenzoïque.

8. Conjugués selon les revendications 1 à 4, dans lesquels ledit composé A est choisi parmi le groupe constitué d'acide acétique, d'acide butyrique, d'alanine, de glycine et de peptides contenant de l'alanine et/ou de la glycine.

9. Conjugués selon les revendications 1 à 8 salifiés à l'aide d'un métal choisi parmi le groupe constitué de métaux alcalins, de métaux alcalino-terreux et de métaux de transition.

10. Conjugués selon la revendication 9 salifiés à l'aide d'un métal choisi parmi le groupe constitué de Na⁺, K⁺, Ca⁺⁺, Mg⁺⁺, Cu⁺⁺, Zn⁺⁺, Ag⁺⁺, Au⁺⁺ et CO⁺⁺.

11. Conjugués selon les revendications 1 à 10, dans lesquels les groupes hydroxyle secondaires de l'acide hyaluronique sont dérivatisés pour former un groupe choisi parmi le groupe constitué de -OR, -OCOR, -SO₂H, -OPO₃H₂, -O-CO-(CH₂)ₙ-COOH et -O-(CH₂)ₙ-OCOR, dans lequel n est compris entre 1 et 4 et R est un alkyle en C₁-C₁₀.

12. Conjugués selon les revendications 1 à 10, dans lesquels les groupes hydroxyle secondaires de l'acide hyaluronique sont dérivatisés pour former un groupe choisi parmi -NH₂ et -NHCOCH₃.

13. Utilisation de conjugués selon les revendications 1 à 12 dans la fabrication d'un médicament pour le traitement de pathologies **caractérisées par** une hyperprolifération cellulaire.

14. Utilisation selon la revendication 13, dans laquelle lesdites pathologies sont choisies parmi le groupe constitué de tumeurs, d'affections cutanées, de psoriasis, de pathologies inflammatoires et d'arthrites rhumatoïdes.

15. Compositions pharmaceutiques contenant des conjugués tels que revendiqués dans les revendications 1 à 12 en mélange avec des excipients et/ou diluants pharmaceutiquement acceptables.

16. Procédé pour la préparation de conjugués tels que revendiqués dans les revendications 1 à 12.

17. Procédé selon la revendication 16, lequel comporte les étapes suivantes à exécuter dans l'ordre indiqué :
(a) chloration des groupes hydroxyle primaires des unités N-acétyle-D-glucosamine de l'acide hyaluronique sous forme libre ou de sel,
(b) formation de liaisons ester entre l'acide hyaluronique chloré et les groupes carboxyle du composé de formule (I) par déplacement d'atomes de chlore,
(c) formation de liaisons ester entre le produit de l'étape (b) et le composé A par déplacement des atomes de chlore résiduels à l'aide d'un nucléophile acyle adapté du composé A.

18. Procédé selon la revendication 17, dans lequel le réactif utilisé pour la chloration est le chlorure de méthanesulfonyle dans le N,N-diméthylformamide.

19. Procédé pour obtenir des conjugués entre l'acide hyaluronique et un composé de formule (I) : 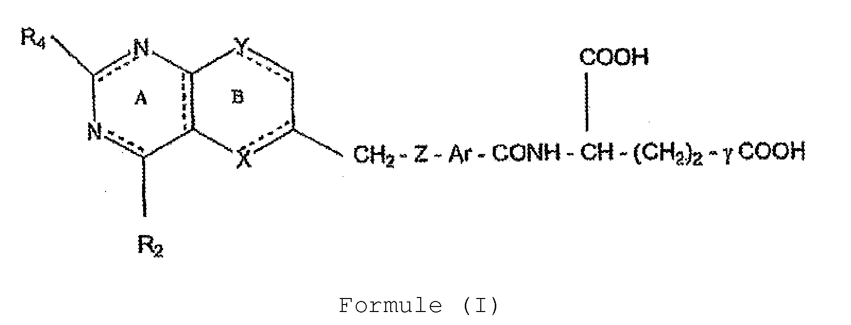 dans laquelle :
R₂ et R₄ représentent : -NH₂, -OH, -OCH₃, alkyle en C₁-C₅,=O,
X et Y représentent -C(R₅)=, -CH(R₅)-, -NH-, -N=, R₅ représentant : H, alkyle en C₁-C₅,
Z représente -CH(R₁₀)-, -N(R₁₀)-, -O-,
R₁₀ représente : -H, un alkyle en C₁-C₅, un alcényle en C₁-C₅, un alcynyle en C₁-C₅, un noyau hétérocyclique à 5 ou 6 chaînons avec 1 à 3 hétéroatomes choisis parmi le groupe constitué de l'azote, du soufre et de l'oxygène,
Ar représente : un groupe 1,4-phényle, un groupe 1,4-phényle condensé avec un ou plusieurs noyaux aromatiques à 5 ou 6 chaînons, un groupe 1,4-phényle condensé avec un ou plusieurs hétérocycles à 5 ou 6 chaînons, lesdits groupes étant éventuellement substitué par R₂,
les noyaux A et B sont aromatiques ou non aromatiques,
**caractérisé en qu'**ils contiennent moins de 3 % p/p de chlore résiduel chimiquement lié à la chaîne polymérique de l'acide hyaluronique,
ledit procédé comportant les étapes suivantes, exécutées dans l'ordre indiqué :
(a) chloration des groupes hydroxyle primaires des unités N-acétyle-D-glucosamine de l'acide hyaluronique sous forme libre ou de sel, et
(b) formation de liaisons ester entre l'acide hyaluronique chloré et les groupes carboxyle du composé de formule (I) par déplacement des atomes de chlore.

20. Procédé pour obtenir des conjugués tels que revendiqués dans la revendication 19, comportant les étapes suivantes, exécutées dans l'ordre indique :
(a) chloration des groupes hydroxyle primaires des unités N-acétyle-D-glucosamine de l'acide hyaluronique sous forme libre ou de sel,
(b) formation de liaisons ester entre l'acide hyaluronique chloré et les groupes carboxyle du composé de formule (I) par déplacement d'atomes de chlore,
(c) déplacement des atomes de chlore résiduels du produit de l'étape (b) par un nucléophile acyle adapté et formation de liaisons ester, et
(d) désacylation sélective des conjugués obtenus à l'étape (c).
